# FASCICULE DE BREVET EUROPEEN

(11) **EP 0 707 644 B1**
(45) Date de publication et mention de la délivrance du brevet: **15.09.2004**
(21) Numéro de dépôt: 94920987.8
(22) Date de dépôt: 27.06.1994
(51) Int. Cl.: C12N 15/11, C07K 14/82, C12N 15/86, A61K 31/70, A61K 35/76

(54) **UTILISATION DE COMPOSITIONS PHARMACEUTIQUES POUR LE TRAITEMENT DES MALADIES NEURODEGENERATIVES**
VERWENDUNG PHARMAZEUTISCHER ZUSAMMENSETZUNGEN ZUR BEHANDLUNG VON NEURODEGENERATIVEN KRANKHEITEN
USE OF PHARMACEUTICAL COMPOSITIONS FOR THE TREATMENT OF NEURODEGENERATIVE DISEASES

(30) Priorité: 30.06.1993 FR 9307962
(43) Date de publication de la demande: 24.04.1996
(60) Demande divisionnaire: 04006618.5
(73) Titulaire: Aventis Pharma S.A., 92160 Antony (FR)
(72) Inventeur: MALLET, Jacques, F-75013 Paris (FR); REVAH, Frédéric, F-92160 Antony (FR); ROBERT, Jean-Jacques, F-92330 Sceaux (FR)
(74) Mandataire: Becker, Philippe
(86) Numéro de dépôt international: PCT/FR1994/000771
(87) Numéro de publication internationale: WO 1995/001429

(56) Documents cités:
- EP-A- 0 185 573
- WO-A-91/11535
- WO-A-91/18088
- WO-A-92/19732
- ERICKSON, R.P. & IZANT, J.G. : 'Gene regulation: biology of antisense RNA and DNA', 1992; RAVEN PRESS, NEW YORK, USA pages 329-353: ' Platelet-derived growth
- SCIENCE, vol.259, 12 Février 1993, LANCASTER, PA US pages 988 - 990 LE GAL LA SALLE, G. ET AL. 'An adenovirus vector for gene transfer into neurons and glia in the brain' cité dans la demande
- WICKSTROM, E. :'Prospects for antisense nucleic acid therapy of cancer and AIDS', 1991, WILEY-LISS, INC., NEW YORK, USA; pages 83-114:' Antisense fos and jun RNA'
- BIOMEDICINE & PHARMACOTHERAPY, vol.46, no.5-7, 1992, ELSEVIER, AMSTERDAM, NL page 257 SCHLINGENSIEPEN, K & BRYSCH, W. 'Specific inhibition of different jun genes either inhibits or stimulates cell differentiation' & 5th International Congress on Differentiation Therapy, September 2-5, 1992; Villasimius, Italy

### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: Compositions pharmaceutiques et leur utilisation, notamment pour le traitement des maladies neurodegeneratives.
   (iii) NOMBRE DE SEQUENCES: 1
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 25 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 1:
   AGCCGCAAGT GACTCAGCGC GGGGC 25

## Description

La présente invention concerne l'utilisation de composés agissant sur l'interaction entre la séquence SEQ ID n° 1 et le facteur de transcription AP1 pour la préparation d'une composition pharmaceutique destinée au traitement des maladies neurodégénératives.

La présente invention résulte en partie de la mise en évidence que le facteur AP1 constitue un médiateur de la dégénérescence neuronale, et que l'emploi de composés capables d'inhiber l'activité de ce complexe peut permettre de bloquer le processus de mort neuronale. Elle résulte également de la mise en évidence que des acides nucléiques correspondant au site d'interaction d'un facteur de transcription sur l'ADN sont capables d'inhiber au moins en partie l'activité de ce facteur de transcription.

Pour étudier les mécanismes moléculaires de la dégénérescence neuronale, la demanderesse a utilisé comme modèle la toxicité induite par le glutamate. Le glutamate est le principal neurotransmetteur excitateur du système nerveux central. Cependant, une exposition au glutamate pendant des périodes anormalement longues, ou à des concentrations plus élevées que les concentrations physiologiques peut provoquer une toxicité neuronale désignée sous le terme d'excitotoxicité (Olney Adv. Exp. Med. Biol. 203 (1986) 631). De nombreux arguments expérimentaux suggèrent que ce type de toxicité contribue à la dégénérescence neuronale associée à l'ischémie. l'hypoxie, l'hypoglycémie, les attaques épileptiques ou encore aux trauma cérébraux (Choi, J. Neurobiol. 23 (1992) 1261). L'excitotoxicité serait également impliquée dans la pathogénèse de maladies telles que la chorée de Huntington (Young et al., Science 241 (1988) 981) et la maladie d'Alzheimer (Koh et al., Brain Res. 533 (1990) 315; Mattson et al.; J. Neurosci. 12 (1992) 376).

Plus précisément, la demanderesse a étudié la mort induite par le glutamate sur des neurones corticaux de rat embryonnaire, en culture primaire. De précédents travaux avaient permis de montrer que la liaision du glutamate sur ses récepteurs membranaires provoquait la dépolarisation de la membrane cellulaire et l'augmentation de calcium intracellulaire, qui conduit à l'activation d'une cascade de second messagers impliquant plusieurs familles d'enzymes. La demanderesse a étudié au niveau génétique la modulation des gènes de réponse précoce codant pour des facteurs de transcription qui, interagissant à leur tour avec des séquences régulatrices de certains gènes, vont activer ou réprimer leur expression. Plus particulièrement, la demanderesse a pu montrer de manière surprenante que l'exposition de cellules corticales au glutamate ou à d'autres excitotoxines telles que le NMDA, provoque l'augmentation du nombre de complexes protéiques susceptibles de se lier à la séquence génomique désignée "12-O-tétradécanoylphorbol 13-acetate-responsive element", soit en abrégé, TRE [Schönthal et al., Cell 54 (1988) 325; Lucibello et al., Oncogene 3 (1988) 43; Angel et al., Cell 49 (1987) 729; Bohmann et al., Science 238 (1988) 1386]. La séquence de la région TRE est représentée sur la SEQ ID n° 1. De plus, la demanderesse a également montré qu'en séquestrant les facteurs susceptibles de se lier sur la séquence SEQ ID n° 1, les cellules corticales sont sauvées de la mort induite par certaines gammes de concentrations de glutamate. Ceci démontre que la séquence SEQ ID n° 1 joue un role de médiateur de la dégénérescence neuronale, observation qui n'a jamais été rapportée dans l'art antérieur. La séquence SEQ ID n° 1 et l'ensemble des facteurs de transcription susceptibles d'interagir avec elle constitue donc une nouvelle cible pharmacologique dans le traitement des processus neurodégénératifs. L'invention réside donc en partie dans l'utilisation de composés capables de bloquer l'activité de la séquence SEQ ID n° 1 pour le traitement des maladies neurodégénératives.

Un premier objet de la présente invention réside donc dans l'utilisation d'un composé inhibant l'interaction entre la séquence SEQ ID n° 1 et le facteur de transcription AP1 qui interagit avec elle pour la préparation d'une composition pharmaceutique destinée au traitement et/ou à la prévention des maladies neurodégénératives.

Les composés inhibant au moins partiellement l'interaction entre la séquence SEQ ID n° 1 et le facteur de transcription AP1 au sens de la présente invention peuvent être des composés agissant par exemple sur la liaison de ces facteurs à la séquence SEQ ID n° 1.

Les facteurs de transcription qui interagissent avec la séquence SEQ ID n° 1 ont été regroupés sous le terme générique AP1 (Landschulz et al., Science 240 (1988) 1759). Il s'agit de dimères dont les composants élémentaires sont liés entre eux par un motif structurel de "fermeture à glissière à leucine". Typiquement, un des composants appartient à la famille des proto-oncogènes Fos (famille comprenant notamment les protéines Fos, Fra1, Fra2 et FosB) et l'autre à la famille Jun (famille comprenant notamment les protéines Jun, JunB et JunD). A titre d'exemple, la protéine Fos est composée de 380 acides aminés, Jun de 354 (revue dans Mc Mahon et Monroe, FASEB J. 6 (1992) 2707).

Parmi les composés agissant sur la synthèse des facteurs se liant à la séquence SEQ ID n° 1, on peut citer les séquences nucléotidiques antisens dirigées contre ces facteurs ou leurs composants, qui inhibent la traduction des gènes correspondants. Ainsi, dans le cas de Fos et de Jun, on peut utiliser les antisens décrits par Holt et al. (PNAS 83 (1986) 4794) ou par Kovary et Bravo (Mol.Cell.Biol. 11 (1991) 4466). D'autres composés agissant sur la régulation de l'expression des gènes de ces facteurs peuvent également être mentionnés tels que notamment le curcumin (Huang et al., PNAS 88 (1991) 5292), la 2-amino-purine (Zinn et al., Science 240 (1988) 210) ou encore l'héparine (Wright et al., PNAS 86 (1989) 3199), composés qui sont capables d'agir sur la synthèse de Fos et de Jun.

Une autre classe de composés utilisables regroupe les composés agissant sur les mécanismes moléculaires de modulation de l'activité des facteurs de transcription interagissant avec la séquence SEQ ID n° 1. Ces mécanismes impliquent notamment des étapes de multimérisation, phosphorylation-déphosphorylation, etc. En ce qui concerne le facteur AP1, celui-ci pour être actif doit être phosphorylé, phosphorylation catalysée par la protéine kinase C ou la protéine kinase A (revue dans Mc Mahon et Monroe précitée), ainsi que par une protéine kinase ADN dépendante (Bannister et al., Nucleic Acids Res. 21 (1993) 1289). Parmi les composés agissant sur la phosphorylation d'AP1 au sens de l'invention, on peut donc citer les composés capables d'inhiber au moins partiellement l'activité de ces protéines kinases.

Finalement, comme indiqué plus haut, les composés au sens de la présente invention sont ceux capables d'inhiber au moins partiellement l'interaction entre le facteur de transcription AP1 et la séquence SEQ ID n° 1. De tels composés peuvent être notamment des antagonistes des facteurs de transcription, ou des protéines capables d'interagir avec les facteurs de transcription ou leurs composants ou la séquence ID n° 1 et de moduler ainsi l'activité de laison entre ces facteurs et la séquence SEQ ID n° 1. A cet égard, on peut citer les protéines de la famille CREB (Hai et Curran, PNAS 88 (1991) 3720), LRF-1 (Hsu et al., PNAS 88 (1991) 3511), IP-1 (Auwerx et Sassone-Corsi, Cell 64 (1991) 983), ou des récepteurs aux stéroïdes (revue dans Miner et Yamamoto, Trends in Biochem. Sci. 16 (1991) 423). Les membres de la famille CREB interagissent avec les monomères de la famille Fos ou Jun. Les différents hétérodimères ont des spécificités de liaison différentes. Ainsi, les dimères croisés Fos-CREB ou Jun-CREB peuvent se lier sur le site TRE mais également sur le site CRE (site de liaison des protéines CREB sur l'ADN), selon la composition du dimère. Plus précisément, ils présentent généralement une liaison préférentielle au site CRE (Hai et Curran précitée). La dimérisation d'un membre de la famille CREB avec un membre de la famille Fos ou Jun induit donc une modulation du nombre de facteurs liés à la séquence SEQ ID n° 1. La protéine LRF-1 forme des dimères avec c-Jun et Jun-B. Ces dimères sont capables de se lier in vitro au site CRE et de ce fait, d'interférer avec le nombre de facteurs liés à la séquence SEQ ID n° 1 (Hsu et al. précitée). IP-1 est un facteur endogène retrouvé dans le noyau et le cytoplasme de plusieurs types cellulaires. IP-1 bloque de manière spécifique la liaison à l'ADN des facteurs AP1. C'est une protéine de 30 à 40 kDa, qui exerce son activité lorsqu'elle est sous forme phosphorylée (Auwerx et Sassone-Corsi précitée). En ce qui concerne les récepteurs aux stéroïdes (glucocorticoïdes, minéralocorticoïdes, progestérone, androgènes, etc), ce sont des récepteurs nucléaires qui appartiennent à la famille des facteurs de transcription à doigt de zinc. Ils possèdent des sites d'interaction avec l'ADN différents de la SEQ ID n° 1. Toutefois, il existe des éléments de réponse composite dans les régions régulatrices des gènes, au niveau desquels les membres de la famille des récepteurs aux stéroïdes et les membres de la famille des facteurs de transcription à fermeture à glissière à leucine (AP1, CREB, LRF-1, etc) peuvent interagir (via des interactions protéine-protéine ou protéine-ADN) pour produire des effets de régulation, qui n'existent pas en présence d'un seul de ces facteurs. Pour ces raisons, l'ensemble de ces protéines étant capable d'interagir directement ou indirectement avec les facteurs AP1 et/ou la séquence SEQ ID n° 1, elles peuvent être utilisées dans le cadre de la présente invention pour inhiber le processus de dégénérescence neuronale. Ces protéines peuvent être utilisées telles quelles ou sous forme de constructions génétiques capables d'exprimer in vivo ces protéines. D'autres composés capables d'inhiber au moins partiellement l'interaction entre les facteurs de transcription et la séquence SEQ ID n° 1 sont constitués par des acides nucléiques double brin reproduisant le site de liaison des facteurs de transcription à l'ADN, c'est-à-dire la séquence SEQ ID n° 1, ou tout variant actif de celle-ci. La demanderesse a en effet montré que de tels acides nucléiques étaient capables de complexer les facteurs de transcription (et en particulier le facteur AP1) présents dans les cellules, de les empecher de se fixer sur leurs sites endogènes, et ainsi, de bloquer leur activité transcriptionnelle.

Dans un mode préféré, le composé utilisé dans le cadre de la présente invention est un acide nucléique double brin comprenant tout ou partie de la séquence SEQ ID n° 1 ou d'un variant actif de celle-ci.

Le terme variant actif désigne au sens de l'invention tout variant de la séquence SEQ ID n° 1 ayant conservé les propriétés de fixation au facteur AP1. De tels variants peuvent être obtenus par mutation, délétion, substitution et/ou addition de bases sur la séquence SEQ ID n° 1, puis vérification in vitro de l'activité de liaison comme décrit dans les exemples 1 et 2.

Cet acide nucléique double brin peut être utilisé tel quel, par exemple après injection à l'homme ou l'animal, pour induire une protection ou traiter la dégénérescence neuronale. En particulier, il peut être injecté sous forme d'ADN nu selon la technique décrite dans la demande WO 90/11092. Il peut également être administré sous forme complexée, par exemple avec du DEAE-dextran (Pagano et al., J.Virol. 1 (1967) 891), avec des protéines nucléaires (Kaneda et al., Science 243 (1989) 375), avec des lipides (Felgner et al., PNAS 84 (1987) 7413), sous forme de liposomes (Fraley et al., J.Biol.Chem. 255 (1980) 10431), etc.

Préférentiellement, l'acide nucléique double brin utilisé dans le cadre de l'invention fait partie d'un vecteur. L'emploi d'un tel vecteur permet en effet d'améliorer l'administration de l'acide nucléique dans les cellules à traiter, et également d'augmenter sa stabilité dans lesdites cellules, ce qui permet d'obtenir un effet inhibiteur durable. De plus, il est possible d'introduire plusieurs séquences d'acide nucléique dans un même vecteur, ce qui augmente également l'efficacité du traitement.

Le vecteur utilisé peut être d'origine diverses, dès lors qu'il est capable de transformer les cellules animales, de préférence les cellules nerveuses humaines. Dans un mode préféré de mise en oeuvre de l'invention, on utilise un vecteur viral, qui peut être choisi parmi les adénovirus, les rétrovirus, les virus adéno-associés (AAV), le virus de l'herpès, etc.

A cet égard, la présente invention utilise tout virus recombinant comprenant, inséré dans son génome, un acide nucléique double brin correspondant au site d'interaction d'un facteur de transcription sur l'ADN. La présente invention démontre en effet la possibilité de traiter certaines pathologies résultant de l'expression de certains gènes non pas en affectant directement le ou les gènes impliqués, ni leurs ARN messagers, ni encore le produit de leur traduction, mais en affectant l'activité des facteurs de transcription responsables de leur expression. Cette approche permet de contrôler l'expression de nombreux gènes, tels que par exemple celle du gène codant pour les différentes isoformes du précurseur de la protéine amyloïde (APP), impliquée dans la pathogénèse de l'Alzheimer. Ce gène comporte en effet 2 sites de liaison d'un facteur de transcription (AP1) à moins de 400 pb en amont du site de démarrage de la transcription (Lee et al., Nature 325 (1987) 368). De même, le promoteur du gène de la tyrosine hydroxylase, enzyme clé de la synthèse des cathécolamines, impliquée dans la maladie de Parkinson, contient un site de liaison du facteur AP1 (Icard-Liepkans et al., J. Neurosc. Res. 32 (1992) 290). Un autre exemple concerne le gène du NGF qui possède également un site de liaison d'un facteur de transcription (S D'Mello et Heinrich, Mol Brain Res. 11 (1991) 255).

Le virus recombinant peut être choisi parmi les adénovirus, les rétrovirus, les virus adéno-associé, etc. De préférence, il s'agit d'un virus capable d'infecter les cellules nerveuses, tel que notamment un adénovirus. Des vecteurs dérivés des adénovirus, des rétrovirus, ou des AAV incorporant des séquences d'acides nucléiques hétérologues ont été décrits dans la littérature [Akli et al., Nature Genetics 3 (1993) 224; Stratford-Perricaudet et al., Human Gene Therapy 1 (1990) 241; EP 185 573, Levrero et al., Gene 101 (1991) 195; Le Gal la Salle et al., Science 259 (1993) 988; Roemer et Friedmann, Eur. J. Biochem. 208 (1992) 211; Dobson et al., Neuron 5 (1990) 353; Chiocca et al., New Biol. 2 (1990) 739; Miyanohara et al., New Biol. 4 (1992) 238; WO91/18088].

Les virus recombinants tels que définis ci-dessus comprennent un acide nucléique double brin correspondant au site d'interaction du facteur AP1 sur l'ADN. Encore plus préférentiellement, l'acide nucléique double brin comprend tout ou partie de la séquence SEQ ID n° 1 ou d'un variant actif de celle-ci.

Avantageusement, le virus recombinant est un virus défectif. Le terme "virus défectif" désigne un virus incapable de se répliquer dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par l'acide nucléique double brin. Préférentiellement, le virus défectif conserve néanmoins les séquences de son génome qui sont nécessaires à l'encapsidation des particules virales.

D est particulièrement avantageux d'utiliser les séquences nucléiques de l'invention sous forme incorporée à un adénovirus recombinant défectif.

Il existe en effet différents sérotypes d'adénovirus, dont la structure et les propriétés varient quelque peu, mais qui ne sont pas pathogènes pour l'homme, et notamment les sujets non immuno-déprimés. Par ailleurs, ces virus ne s'intègrent pas dans le génome des cellules qu'ils infectent, et peuvent incorporer des fragments importants d'ADN exogène. Parmi les différents sérotypes, on préfère utiliser dans le cadre de la présente invention les adénovirus de type 2 ou 5 (Ad 2 ou Ad 5). Dans le cas des adénovirus Ad 5, les séquences nécessaires à la réplication sont les régions E1A et E1B.

Par ailleurs, la faible taille des acides nucléiques correspondant au site de liaison à l'ADN d'un facteur de transcription selon l'invention permet de manière avantageuse d'incorporer simultanément, dans un même vecteur, plusieurs acides nucléiques, identiques (destinés à bloquer le même facteur de transcription) ou différents (destinés à bloquer des facteurs de transcription différents). Un mode de réalisation particulier de l'invention consiste donc dans l'utilisation d'un vecteur, notamment viral, comprenant au moins deux acides nucléiques tels que définis ci-dessus.

Les virus recombinants défectifs peuvent être préparés par recombinaison homologue entre un virus défectif et un plasmide portant entre autre la séquence d'acides nucléiques telle que définie ci-avant (Levrero et al., Gene 101 (1991) 195; Graham, EMBO J. 3(12) (1984) 2917). La recombinaison homologue se produit après co-transfection desdits virus et plasmide dans une lignée cellulaire appropriée. La lignée cellulaire utilisée doit de préférence (i) être transformable par lesdits éléments, et (ii), comporter les séquences capables de complémenter la partie du génome du virus défectif, de préférence sous forme intégrée pour éviter les risques de recombinaison. A titre d'exemple de lignée utilisable pour la préparation d'adénovirus recombinants défectifs, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12%). A titre d'exemple de lignée utilisable pour la préparation de rétrovirus recombinants défectifs, on peut mentionner la lignée CRIP (Danos et Mulligan, PNAS 85 (1988) 6460).

Ensuite, les virus qui se sont multipliés sont récupérés et purifiés selon les techniques classiques de biologie moléculaire.

La présente invention a également pour objet l'utilisation d'une composition pharmaceutique comprenant au moins un virus recombinant ou un acide nucléique correspondant au site d'interaction d'un facteur de transcription sur l'ADN tels que définis ci-dessus. Plus préférentiellement, l'invention concerne l'utilisation d'une composition pharmaceutique comprenant au moins un acide nucléique double brin comprenant tout ou partie de la séquence SEQ ID n° 1 ou d'un variant actif de celle-ci, ou un virus recombinant comprenant un tel acide nucléique.

Les compositions pharmaceutiques peuvent être formulées en vue d'une administration par voie topique, orale, parentérale, intranasale, intraveineuse, intramusculaire, sous-cutanée, intraoculaire, etc.

Préférentiellement, les compositions pharmaceutiques contiennent des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses d'acides nucléiques (séquence ou vecteur) utilisées pour l'administration peuvent être adaptées en fonction de différents paramètres, et notamment en fonction du mode d'administration utilisé, de la pathologie concernée, de l'acide nucléique à exprimer, ou encore de la durée du traitement recherchée. D'une manière générale, concernant les virus recombinants ceux-ci sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

De telles compositions pharmaceutiques peuvent être utilisées chez l'homme, pour le traitement et/ou la prévention des maladies neurodégénératives, et en particulier, pour le traitement et/ou la prévention de la dégénérescence neuronale associée à l'ischémie, l'hypoxie, l'hypoglycémie, les attaques épileptiques ou encore aux trauma cérébraux, ou pour le traitement et/ou la prévention de la chorée de Huntington ou de la maladie d'Alzheimer.

La présente invention sera plus complètement décrite à l'aide des exemples qui suivent, qui doivent être considérés comme illustratifs et non limitatifs.

### Légende des figures

### SEQ ID n° 1 : Séquence du fragment TRE.

Figure 1 : Mise en évidence par mesure de la liaison d'ouabaïne de l'inhibition par la séquence SEQ ID n° 1 de la mort cellulaire induite par le glutamate. Cet effet inhibiteur n'est pas observé en présence de la séquence SEQ ID n° 1 mutée aux positions 10 (T -> G) et 15 (C -> T).
Figure 2 : Mise en évidence par mesure du LDH libéré dans le milieu extracellulaire de l'inhibition par la séquence SEQ ID n° 1 de la mort cellulaire induite par le glutamate. Cet effet inhibiteur n'est pas observé en présence de la séquence SEQ ID n° 1 mutée aux positions 10 (T -> G) et 15 (C -> T).
Figure 3 : Mise en évidence par coloration au bleu trypan de l'inhibition par la séquence SEQ ID n° 1 de la mort cellulaire induite par le glutamate.

### Techniques générales de clonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Multis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### EXEMPLES

### Exemple 1 : Mise en évidence de complexes liant la séquence SEQ ID n° 1 après exposition des cellules corticales au glutamate.

Cet exemple démontre une augmentation du nombre de complexes intracellulaires capables de lier la séquence SEQ ID n° 1, après exposition des cellules corticales au glutamate. Cette démonstration a été réalisée en utilisant la technique de gel-retard (Lassar et al., Cell 66 (1991) 305).

Pour cela, les cellules de cortex de rat Wistar embryonnaire (E17) ont été isolées selon la méthode de Dichter (Brain Res. 149 (1978) 279), mises en culture sur boites 6 puits (35 mn; densité 6.10⁵ cellules/boite) Costar, dans du milieu DMEM (Dulbecco's Modified Eagle Medium) contenant 10 µg/ml insuline, 10 µg/ml transferrine, 10 ng/ml selenite de sodium, 10 nM progestérone, 1 nM triiodothyronine, et conservées dans une étuve (37°C, 5 % CO2).

Après 4 à 6 jours en culture, du glutamate est ajouté aux cellules. Dix minutes à 48 heures plus tard, les extraits nucléaires des cellules ont été obtenus selon la technique décrite par Lassar et al. précitée. Une sonde radioactive correspondant à la séquence SEQ ID n° 1 a été préparée selon l'exemple 2, puis par marquage au phosphore 32 selon la technique décrite par Maniatis (activité spécifique typique : 50000 cpm/ng). 0,2 ng le la sonde ainsi obtenue sont ensuite incubés avec 0,5 à 2 µg d'extrait nucléaire, en présence de 500 ng de poly D(IC)(IC) (Pharmacia) pour diminuer la liaison non spécifique. La réaction a lieu dans la glace, pendant 10 min. Les complexes formés sont alors révélés par électrophorèse sur gel d'acrylamide 5%. La présence de bandes radioactives (mises en évidence par autoradiographie) démontre que le glutamate a induit l'expression cellulaire de complexes capables de lier la SEQ ID n° 1.

### Exemple 2 : Synthèse de la séquence SEQ ID n° 1 et d'un variant inactif.

Un acide nucléique double brin correspondant à la séquence SEQ ID n° 1 a été synthétisé au moyen d'un synthétiseur automatique de nucléotides (Maniatis).

Un mutant inactif de cet acide nucléique a été préparé selon le même protocole. Ce mutant inactif présente, par rapport à la séquence SEQ ID n° 1 des mutations aux positions 10 (T -> G) et 15 (C -> T). L'absence d'activité de ce mutant a été démontrée dans les mêmes conditions que pour l'exemple 1, en utilisant ce mutant, après marquage radioactif, comme sonde. L'inactivité de ce mutant est montrée par l'absence de changement de son profil de liaison aux extraits cellulaires, comparé à la SEQ ID n° 1, après culture en présence de glutamate.

La capacité de la séquence SEQ ID n° 1 d'inhiber la mort induite par le glutamate a été déterminée par mesure de trois paramètres : la liaison d'ouabaïne tritiée aux neurones en culture, la détection de la lactate déshydrogénase dans le milieu extracellulaire, et par comptage des cellules après coloration au bleu tryptan.

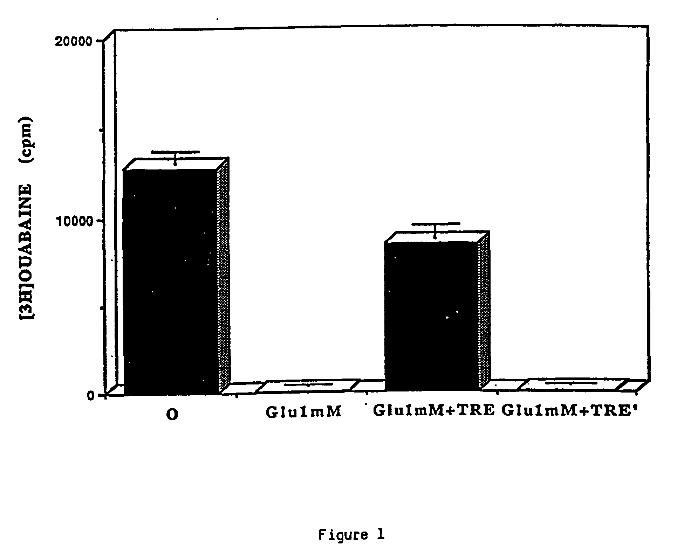

### Exemple 3 : Mesure de la liaison d'ouabaïne tritiée aux neurones en culture (figure 1).

L'ouabaïne est un ligand de l'ATPase membranaire. Elle ne se fixe que sur les neurones vivants. Ainsi, plus la quantité d'ouabaïne fixée est élevée, plus le nombre de cellules vivantes est grand.

Les cellules de cortex de rat Wistar embryonnaire (E 17) ont été isolées selon la méthode de Dichter (Brain Res. 149 (1978) 279), mises en culture sur boites 6 puits (35 mn; densité 6.10⁵ cellules/boite) ou 24 puits (16 mn; densité 3.10⁵ cellules/boite) Costar, dans du milieu DMEM (Dulbecco's Modified Eagle Medium) contenant 10 µg/ml insuline, 10 µg/ml transferrine, 10 ng/ml selenite de sodium, 10 nM progestérone, 1 nM triiodothyronine, et conservées dans une étuve (37°C, 5 % CO2). Après 4 ou 5 jours de culture, 2µM d'acide nucléique synthétique de séquence SEQ ID n° 1 ou de son variant inactif (Cf exemple 2) ont été ajoutés aux boites de culture. Après 15 heures d'incubation, le glutamate (1 mM) a été ajouté. La liaison d'ouabaïne tritiée sur les cultures a été évaluée 24 heures après l'addition du glutamate, selon le protocole décrit par Markwell et al., Brain Res. 538 (1991) 1).

Les résultats obtenus sont présentés sur la figure 1. Ils montrent clairement que le glutamate induit une perte de liaison de l'ouabaïne aux cellules, que cette perte est inhibée en présence de l'acide nucléique synthétique de séquence SEQ ID n° 1, mais pas en présence du variant muté inactif.

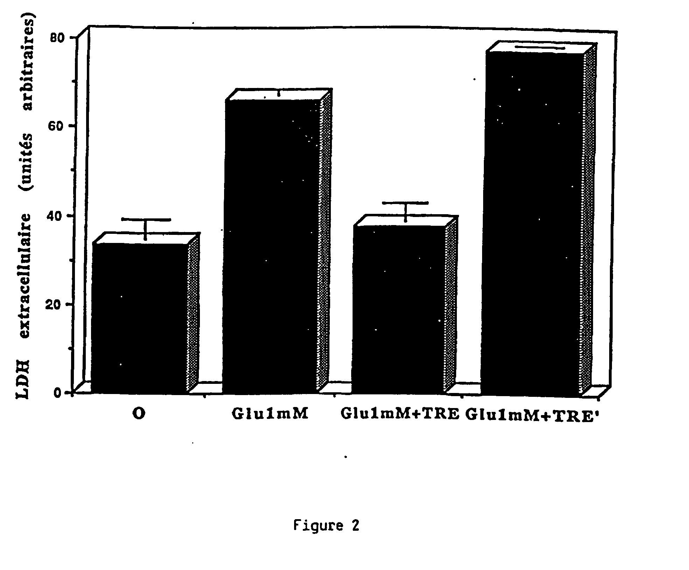

### Exemple 4 : Mesure du relargage de la lactate déshydrogénase dans le milieu extracellulaire (figure 2).

La lactate déshydrogénase (LDH) est une enzyme cytoplasmique qui est relarguée dans le milieu extracellulaire par les cellules mourantes. Ainsi, plus la quantité de LDH relarguée est élevée, plus le nombre de cellules mourantes est grand.

Les cellules ont été cultivées dans les mêmes conditions que dans l'exemple 3. L'activité LDH présente dans le milieu extracellulaire a été dosée 24 heures après l'addition du glutamate, selon la méthode de Berger et Brodia (procédure Sigma 500).

Les résultats obtenus sont présentés sur la figure 2. Ils montrent clairement que le glutamate induit une augmentation du niveau extracellulaire de LDH, que cette augmentation est inhibée en présence de l'acide nucléique synthétique de séquence SEQ ID n° 1, mais pas en présence du variant muté inactif.

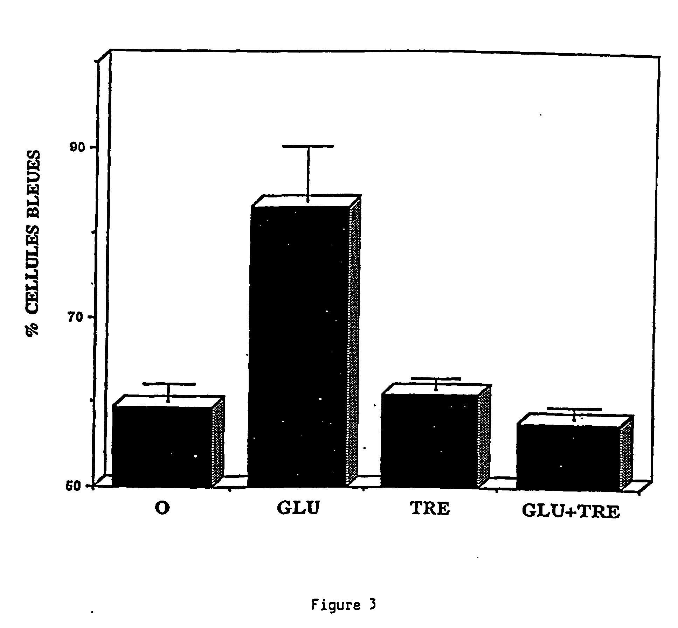

### Exemple 5 : Comptage des cellules après coloration au bleu trypan (figure 3).

Le bleu trypan est un colorant vital qui entre dans les cellules mortes, qui deviennent "bleues", tant dis qu'il est exclu des cellules vivantes qui restent "blanches". Ainsi, plus la quantité de cellules "bleues" est élevée, plus le nombre de cellules mortes est grand.

Les cellules ont été cultivées dans les mêmes conditions que dans l'exemple 3. La concentration de glutamate utilisée dans cet exemple est de 5 mM. En fin d'incubation, le Bleu Trypan a été ajouté à la concentration de 0,02 % et les cellules ont été replacées à l'étuve pendant 10 minutes. Chaque puits (6 puits par condition) a ensuite été photographié 5 fois à l'aide d'un microscope Zeiss 135M. Le nombre de cellules bleues et blanches a ensuite été compté en aveugle sur chacune des photos.

Les résultats obtenus sont présentés sur la figure 3. Ils montrent clairement que le glutamate induit une mort cellulaire importante, et que cette mort cellulaire est inhibée en présence de l'acide nucléique synthétique de séquence SEQ ID n° 1.

L'ensemble de ces résultats démontre clairement la capacité de l'acide nucléique utilisé dans le cadre de l'invention d'inhiber la mort neuronale induite par le glutamate.

## Revendications

1. Utilisation d'un composé inhibant la liaison du facteur AP1 à la séquence SEQ ID n° 1 pour la préparation d'une composition pharmaceutique destinée au traitement et/ou à la prévention des maladies neurodégénératives.

2. Utilisation selon la revendication 1, d'un acide nucléique double brin comprenant la séquence SEQ ID n° 1 ou un variant actif de celle-ci ayant les propriétés de fixation au facteur AP1.

3. Utilisation selon la revendication 2 **caractérisée en ce que** l'acide nucléique double brin fait partie d'un vecteur.

4. Utilisation selon l'une des revendications 2 et 3 **caractérisée en ce que** l'acide nucléique double brin fait partie d'un vecteur viral.

5. Utilisation selon l'une des revendications 3 et 4 **caractérisée en ce que** le vecteur est un adénovirus, un rétrovirus, un virus adéno-associé, ou le virus de l'herpès.

6. Utilisation selon la revendication 5 **caractérisée en ce qu'**il s'agit d'un adénovirus.

7. Utilisation d'un virus recombinant selon l'une des revendications 4 à 6 caractérisée en ce ledit virus comprend plusieurs acides nucléiques double brin, identiques ou différents, correspondant au site d'interaction d'un facteur de transcription sur l'ADN.

8. Utilisation d'un virus recombinant selon l'une des revendications 4 à 6 **caractérisée en ce que** ledit virus est un virus défectif.

## Patentansprüche

1. Verwendung einer Zusammensetzung, die die Bindung des Faktors AP1 an die Sequenz SEQ ID Nr. 1 hemmt zur Herstellung einer pharmazeutischen Zusammensetzung für die Behandlung und/oder Vorbeugung von neurodegenerativen Krankheiten.

2. Verwendung nach Anspruch 1 einer doppelsträngigen Nukleinsäure mit der Sequenz SEQ ID Nr. 1 oder einer aktiven Variante davon mit den Eigenschaften, daß sie sich an den Faktor AP1 anlagert.

3. Verwendung nach Anspruch 2, **dadurch gekennzeichnet, daß** die doppelsträngige Nukleinsäure Bestandteil eines Vektors ist.

4. Verwendung nach Anspruch 2 oder 3, **dadurch gekennzeichnet, daß** die doppelsträngige Nukleinsäure Bestandteil eines viralen Vektors ist.

5. Verwendung nach Anspruch 3 oder 4, **dadurch gekennzeichnet, daß** es sich bei dem Vektor um ein Adenovirus, ein Retrovirus, ein adeno-assoziiertes Virus oder das Herpesvirus handelt.

6. Verwendung nach Anspruch 5, **dadurch gekennzeichnet, daß** es sich um ein Adenovirus handelt.

7. Verwendung eines rekombinanten Virus nach einem der Ansprüche 4 bis 6, **dadurch gekennzeichnet, daß** das Virus mehrere doppelsträngige Nukleinsäuren enthält, die gleich oder verschieden sein können und dem Ort der Wechselwirkung eines Transkriptionsfaktors mit der DNA entsprechen.

8. Verwendung eines rekombinanten Virus nach einem der Ansprüche 4 bis 6, **dadurch gekennzeichnet, daß** es sich bei dem Virus um ein defektives Virus handelt.

## Claims

1. Use of a compound which inhibits the binding of factor AP1 to the sequence SEQ ID No. 1 for the preparation of a pharmaceutical composition intended for the treatment and/or the prevention of neurodegenerative diseases.

2. Use according to Claim 1 of a double-stranded nucleic acid comprising the sequence SEQ ID No. 1 or an active variant thereof having the properties of attaching to factor AP1.

3. Use according to Claim 2, **characterized in that** the double-stranded nucleic acid is part of a vector.

4. Use according to Claim 2 or 3, **characterized in that** the double-stranded nucleic acid is part of a viral vector.

5. Use according to Claim 3 or 4, **characterized in that** the vector is an adenovirus, a retrovirus, an adeno-associated virus or the herpes virus.

6. Use according to Claim 5, **characterized in that** it is an adenovirus.

7. Use of a recombinant virus according to any of Claims 4 to 6, **characterized in that** said virus comprises several double-stranded nucleic acids which are identical or different and which correspond to the interaction site of a transcription factor on DNA.

8. Use of a recombinant virus according to any of Claims 4 to 6, **characterized in that** the virus is a defective virus.
